# EUROPEAN PATENT APPLICATION

(11) **EP 3 871 690 A1**
(43) Date of publication of application: **01.09.2021**
(21) Application number: 20159795.2
(22) Date of filing: 27.02.2020
(51) Int. Cl.: A61K 39/275, C12N 7/02

(54) **METHOD FOR VIRUS PRODUCTION**

(71) Applicant: Technische Hochschule Mittelhessen, 35390 Giessen (DE); Eberhard Karls Universität Tübingen, 72074 Tübingen (DE)
(72) Inventor: Lothert, Keven, 35390 Gießen (DE); Wolff, Michael, 16348 Wandlitz (DE); Pagallies, Felix, 72336 Balingen (DE); Feger, Thomas, 72070 Tübingen (DE); Amann, Ralf, 72070 Tübingen (DE)
(74) Representative: Stumpf, Peter

(57) **Abstract**

The present invention concerns an improved method for the purification of virus from family of poxviridae, for example genus Parapoxvirus, especially ORFV.

The method comprises the following steps:
i) Providing cell culture with cells susceptible for virus from family of poxviridae
ii) Infection of cells from step i) with one species of virus from family of poxviridae to get infected cells
iii) clarification of virus from infected cells from step ii) by filtration, centrifugation, extraction, precipitation or expanded bed adsorption chromatography to remove particles ≥ 1µm and obtain a cell-free virus solution with at least 70% virus recovery
iv) nucleic acid degradation to remove nucleic acids in cell-free virus solution from step iii) with nuclease treatment to get a virus solution with degraded nucleic acid
v) purification of virus from solution of step iv) in at least one chromatographic step with steric exclusion chromatography (SXP) so that the virus is in the product fraction with virus capture yielding above 50% compared to the amount of virus in solution of step iii).

The improvement includes greater efficiency and higher yields, as well as adaptation to regulatory protocols regarding GMP, purity and quality of EMA for GTMP (= Gene Therapy Medicinal Product). Furthermore, the method can be used as a platform technology to purify virus without major process changes.

## Description

The present invention concerns an improved method for production of virus, in particular virus from family of poxviridae, for example genus Parapoxvirus, especially ORF virus (ORFV). The improvement includes greater efficiency and higher yields, as well as adaptation to regulatory protocols regarding GMP, purity and quality according to regulatory authorities (European Medicines Agency, EMA or Food and Drug Administration, FDA/USFDA) for GTMP (= Gene Therapy Medicinal Product). Poxviridae is a family of viruses. Humans, vertebrates and arthropods serve as natural hosts. There are currently 69 species in this family, divided among 28 genera, which are divided into two subfamilies.

ORFV has a size of 140-300 nm and is an enveloped, complex. double-stranded DNA virus comprising linear GC-rich sequences and a central region delimited by inverted terminal repeat (ITR) regions. The central part of the genome contains genes that are highly conserved within the group of pox viruses and are responsible for viral replication. The inverted terminal repeat regions are less or not conserved, they vary within the group of pox viruses and are responsible for host spectrum, pathogenicity and immune modulation. ORFV is a zoonotic pathogen that infect mainly goats and sheep. The infection is caused by lesions of the skin through which the virus can enter the body. In the body, the virus replicates mainly in the keratinocytes and, through the infiltration of lymphocytes, triggers a self-limiting formation of pustules at the site of virus entry. The lesions heal after 4-6 weeks without scarring. However, there is no long-lasting immune protection, so that a reinfection is possible after only a few weeks.

Virus from family of poxviridae, for example genus Parapoxvirus, especially ORF virus (ORFV) has a variety of properties that make it interesting for the production. Patent application DE102015111756A1 describes a method to produce a recombinant ORFV as vector for medical applications. The production and purification process described in this document is not sufficient for purity and quality according to regulatory authorities and typical protocols from other viruses cannot be used for production of virus from family of poxviridae, for example genus Parapoxvirus, especially ORF virus (ORFV) to reach purity and quality according to regulatory authorities.

It is the aim to use highly purified recombinant or modified virus from family of poxviridae, for example genus Parapoxvirus, especially ORF virus (ORFV) as viral vector to shuttle nucleic acids into humans or animals to prevent or treat human or veterinarian diseases. Such viral vectors are tools used in medicine to introduce genetic material into a target cell for use in patients, that are humans or animals. Often, the genetic material codes for specific and desired genes so that the target cell produces the protein or recombinant proteins.

It is further the aim to use highly purified recombinant or modified virus from family of poxviridae, for example genus Parapoxvirus, especially ORFV to produce recombinant antigens as vaccines against human or veterinarian diseases.

Highly purified virus from family of poxviridae, for example genus Parapoxvirus, especially ORFV are interesting for the production of recombinant vaccines, which have a narrow host spectrum and a very narrow natural host tropism. Thus, an inhibitory pre-immunity against the vector caused by a natural infection is highly unlikely in humans, as is observed with the most common vectors of the vaccinia and adenoviruses. In addition, the exceptionally weak and short-lived ORFV-specific vector immunity allows very effective booster with ORFV-based vaccines directed against other pathogens.

In the recent years ORFV-particles have become an interesting opportunity for gene transfer, vaccination and oncolytic therapy, so higher amounts of proper virus particles are needed. But the correct virus production is in this case connected with several problems and a not sufficiently solved task. The aim is the proper virus production, including up- or downstream processing steps for high titer yields of a purity according to regulatory requirements.

To the best of our knowledge, no purification method is described, that allows the processing of cell culture derived virus from family of poxviridae, for example genus Parapoxvirus, especially ORFV with a sufficient virus recovery yield and purity. This is particularly important with regard to the high regulatory demands for purity for vaccines, gene therapy products and oncolytic viruses. Published studies on the ORFV purification are focused on virus extraction from scab material of sheep and goats or from cell culture using density gradient centrifugation. These methods are not economically scalable and often time consuming. Filtration and (density gradient-) centrifugation techniques are commonly applied for a large variety of different viruses and thus are feasible possibilities. However, these often come along with high investment costs, a poor scalability, product losses and -most importantly- an insufficient impurity removal.

### Problem

The task of the present invention is to provide an improved process for the production of virus from family of poxviridae, for example genus Parapoxvirus, especially ORFV in cell culture with a better virus recovery yield, higher efficiency and purity according to the claims, that fulfills the high regulatory demands for purity for vaccines and virus for gene therapy products.

### Solution

This task is solved according to the invention by an improved method for production of virus from family of poxviridae comprising the following steps:
i) Providing cell culture with cells susceptible for virus from family of poxviridae
ii) Infection of cells from step i) with one species of virus from family of poxviridae to get infected cells
iii) clarification of virus from infected cells from step ii) by filtration, centrifugation, extraction, precipitation or expanded bed adsorption chromatography to remove particles ≥ 1µm and obtain a cell-free virus solution with at least 70% virus recovery
iv) nucleic acid degradation to remove nucleic acids in cell-free virus solution from step iii) with nuclease treatment to get a virus solution with degraded nucleic acid
v) purification of virus from solution of step iv) in at least one chromatographic step with steric exclusion chromatography (SXP) so that the virus is in the product fraction with virus capture yielding above 50% compared to the amount of virus in solution of step iii).

Virus from family of poxviridae, for example genus Parapoxvirus, especially ORFV produced according to this method is useful as vaccine, gene therapy or oncolytic virus therapy for animals and humans. The unique host immune escape ability obtained by purified virus from family of poxviridae, for example genus Parapoxvirus, especially ORFV has made it one of the important candidates for prevention and treatment of various diseases (including chronic viral diseases, tumor and liver fibrosis). Here we describe an improved method for production of virus from family of poxviridae, for example genus Parapoxvirus, especially ORFV that fulfills the requirements for greater efficiency and higher yields, as well as adaptation to regulatory protocols regarding GMP, purity and quality according to regulatory authorities (European Medicines Agency, EMA or Food and Drug Administration, FDA/USFDA) for GTMP (= Gene Therapy Medicinal Product).

For this we evaluated different chromatographic applications that can be used for virus purification.

According to this invention, virus from family of poxviridae, for example genus Parapoxvirus, especially ORFV means all natural viruses and virus strains falling under this family also including recombinant viruses. Recombinant viruses from family of poxviridae, for example genus Parapoxvirus, especially ORFV carrying for example viral antigens from other viruses e.g. the rabies glycoprotein or antigens typical for Aujeszky disease, Borna disease, Influenza or classical swine fever or antigens typical for other human and veterinarian diseases. Recombinant virus from family of poxviridae, for example genus Parapoxvirus, especially ORFV carrying for example the human CD4 surface markers. CD4 is a cell surface receptor/marker, a glycoprotein found on the surface of immune cells such as T helper cells, monocytes, macrophages and dendritic cells. CD4 is a member of the immunglobulin superfamily. Recombinant virus from family of poxviridae carrying at least one fluorescence gene e.g. the green fluorescence gene (ORFV-GFP), the cherry fluorescence gene (ORFV-Cherry). Other fluorescence genes or more than one fluorescence gene is possible. We provide a method that can be used for large scale virus purification with regard to the respective purity requirements. Among these are membrane-based ion exchange, hydrophobic interaction and pseudo-affinity chromatography applications, as well as the steric exclusion chromatography using cellulose membranes and size exclusion chromatography especially with the Capto™ Core 700 resin (Capto Core). Capto Core 700 is composed of a ligand-activated core and inactive shell. The inactive shell excludes large molecules (cut off ∼ Mr 700 000 Da) from entering the core through the pores of the shell. These larger molecules are collected in the column flow through while smaller impurities bind to the internalized ligands. We present data on recovery and contaminant removal. The best results were achieved using steric exclusion or salt tolerant membrane chromatography for virus from family of poxviridae, for example genus Parapoxvirus, especially ORFV capture yielding above 50% of the infected cells.

In another embodiment we used in step v) a second chromatography step with the size exclusion chromatography especially with the Capto™ Core 700 resin or hydrophobic interaction membrane chromatography so that the virus is in the product fraction with overall virus yielding of >85% of the virus particles compared to the amount of virus in the infected cells of step iii) and an overall recovery of at least 10% and a degree of purity of at least 10% total protein reduction and 10% of the DNA content compared to the infected cells of step iii).

The expression "initial clarification" means the separation of larger process contaminants like cells and cell debris from the sample and is performed preceding any chromatography step.

The expression "first clarification" means a first clarification step with depth filters of a larger pore size between 1-10 µm, e.g. 5 µm, and is part of the initial clarification, to remove cells and larger contaminants.

The expression "second clarification" or "final clarification" means a succeeding clarification step with depth filters of a smaller pore size, for example 0.65 µm, and is part of the initial clarification, to remove cell debris and other larger contaminants. The expression "capture" means a first chromatographic purification, in which the virus is retained (captured), while other process contaminants are removed.

The expression "polishing" means a second chromatographic purification step, to remove remaining traces of impurities and achieve a high virus purity.

The expression "virus capture yielding" means the recovery of the virus after the first chromatography purification step compared to the amount of virus before purification this means compared to the infected cells of step ii).

The expression "overall virus yielding" means the recovery of the virus after all chromatography steps (first and second) compared to the amount of virus before purification this means compared to the infected cells of step ii).

### Embodiments

### Step i) Providing cell culture with cells susceptible for virus from family of poxviridae

For the production virus amplification was done in cell culture or culture with suspension cells. Therefore, eukaryotic cells for example mammalian cells especially commercially available Vero cells were seeded in suitable vessels according to standard procedure and standard material known in the state of the art, for example 20.000 Vero cells per cm². The invention is not limited to Vero cells, because other eukaryotic and mammalian cells can be used as well. But Vero cells are easily obtainable and typically used as host cells for growing viruses and are approved to regulatory protocols regarding GMP.

They are a lineage of cells used in cell cultures. The 'Vero' lineage was isolated from kidney epithelial cells extracted from African green monkey (Chlorocebus sp.). The Vero cell lineage is continuous and aneuploid, meaning that it has an abnormal number of chromosomes. A continuous cell lineage can be replicated through many cycles of division and not become senescent. Vero cells are interferon-deficient; unlike normal mammalian cells, they do not secrete Interferon α or Interferon β when infected with virus from family of poxviridae, for example genus Parapoxvirus, especially ORFV. However, they still have the Interferon α receptor and the Interferon β receptor, so they respond normally when recombinant interferon is added to their culture media.

### Step ii) Infection of cells from step i) with one species of virus from family of poxviridae to get infected cells

The infection occurs with one species of virus or virus strains falling under the family of poxviridae, for example Parapoxvirus, especially ORFV including natural virus and recombinant virus.

Recombinant virus for example carrying viral antigens from other viruses e.g. the rabies glycoprotein or antigens typical for Aujeszky disease virus, Borna disease virus, Influenza virus or classical swine fever virus or antigens typical for other viruses causing human and veterinarian diseases. Recombinant virus for example carrying the human CD4 surface markers. CD4 is a cell surface receptor/marker, a glycoprotein found on the surface of immune cells such as T helper cells, monocytes, macrophages and dendritic cells. CD4 is a member of the immunoglobulin superfamily. Recombinant virus for example carrying at least one fluorescence gene e.g. green fluorescence gene (ORFV-GFP), cherry fluorescence gene (ORFV-Cherry). The fluorescence enables the spatial and temporal distribution of virus to be directly observed. Infection with virus was done as known in the state of the art at a multiplicity of infection (MOI) for example of 0.05. After growing e.g. five days the virus was harvested. The results are shown with ORFV, but in principle are the same with other virus from family of poxviridae, for example genus Parapoxvirus.

### Step iii) Clarification of virus from family of poxviridae from infected cells from step ii)

The clarification of virus from family of poxviridae, for example genus Parapoxvirus, especially ORFV from infected cells was performed to remove particles ≥ 1 µm and to obtain a cell-free solution. This was done by filtration, centrifugation, extraction, precipitation or expanded bed adsorption chromatography. For this the infected cells were disrupted for example by freeze-thawing so that virus is released from the Vero cells. The supernatant was either centrifuged in a reasonable way so that pellet containing disrupted cells and a clear supernatant with virus suspension occurs. Preferred, clarification was done by filtration. Filtration experiments were carried out using a suitable equipment for example the Sartoflow Smart operated by the Bi-opat® MFCS 4 software. Clarification was performed using a sequence of depth filters with decreasing pore sizes between 1-10 µm. For cell and cell debris separation filters with 5 µm pore sizes were used, e.g. employing either Sartopure PP3 MidiCaps or Millistak+® C0HC filters. For final clarification, filters of the same material with 0.65 µm pores were applied, for example Sartopure PP3 capsules or Millistak+® D0HC. Sartopure devices had a membrane surface area of 650 cm² (5 µm pores) and 130 cm² (0.65 µm pores), whereas the Millistak+® filters were applied in the µPod®-format with 23 cm² total surface area. The capacity of the filters was estimated by applying unclarified virus harvest, until a pressure limitation occurred. Clarification performance was evaluated by calculating the Nephelometric turbidity unit (NTU) using NTU = 0.191+926.194∗*A*700. Filtration experiments were performed with different culture harvests.

All subsequent chromatographic purifications were carried out using the same batch of clarified virus harvest. All filtration and screening experiments were done using virus from family of poxviridae, for example genus Parapoxvirus, especially ORFV carrying a green fluorescence gene (ORFV-GFP). Additionally, later process runs were additionally performed with other virus strains falling under the species Parapoxvirus ovis, including recombinant viruses. The results are only shown for ORFV, but in principle are the same with other virus from family of poxviridae, for example genus Parapoxvirus.

### Step iv) nucleic acid degradation to degrade nucleic acids

After clarification the cell-free virus solution was treated with nuclease for nucleic acid degradation to degrade nucleic acids in the solution.

### Step v) purification of virus from family of poxviridae from solution of step iv) in at least one chromatographic step with steric exclusion chromatography (SXP)

The result is that the virus from family of poxviridae, for example genus Parapoxvirus, especially ORFV is in the product fraction with virus capture yielding above 50% compared to the amount of virus in solution of step iii). The product fraction with virus is in one case according to the working procedure of the column the flow through. In another case the product fraction with virus is the eluate of the column. Both variations are possible and resulted in nearly the same result.

In a preferred embodiment step v) includes a second chromatographic step to remove proteins and nucleotides in the virus solution after step iv) with size exclusion chromatography, ion exchange chromatography, hydrophobic interaction chromatography or pseudo-affinity chromatography, so that the virus is in the product fraction with overall virus yielding of >85% of the virus particles compared to the amount of virus in solution of step iii) and an overall recovery of at least 10% and a degree of purity of at least 10% total protein reduction and 10% of the DNA content compared to the amount in solution of step iii).

In a further improvement there is an additional filtration step between SXP and the second chromatography step. This was done e.g. with cross-flow filtration, to concentrate the virus or to re-buffer, so that the virus is in a suitable buffer for the second process step. A filter size was used with cut-offs between 100 and 1000 kDa, so that the virus is retained and buffers, salts and anything smaller can be flushed out. The results are shown only for ORFV, but in principle are the same with other virus from family of poxviridae, for example genus Parapoxvirus.

### Experiments for screening of feasable chromatographic process unit operations

To find the best working conditions for the production of virus from family of poxviridae, for example genus Parapoxvirus, especially ORFV several experiments were performed and results collected. All chromatographic purifications were done using a liquid chromatography system for example an Äkta Pure 25 liquid chromatography system with UV (280 nm, Standard wavelength for these devices, since proteins are very well visible here) and conductivity monitoring at a temperature between 19-23°C. Furthermore, the light scattering signal was measured online for example with a Nano DLS Particle Size Analyzer. All buffers are standard buffers with known content, they were filtered with a filter in particular a 0.2 µm filter and degassed by ultrasonification prior to usage.

The results are shown only for ORFV, but in principle are the same with other virus from family of poxviridae, for example genus Parapoxvirus.

### Steric exclusion chromatography (SXC)

The SXC was performed exclusively as capture step. Regenerated cellulose membranes with a nominal pore size of 1 µm were applied as stationary phase. The membranes were punched to disc of 13 or 24 mm and assembled into stainless steel filter holders with 13 mm or 25 mm diameter respectively. For each device 10 membrane layers were stacked giving a total surface area of ∼13.3 cm² for the 13 mm device and 45.2 cm² for the 24 mm membranes.

At first the membrane stack was equilibrated using an equilibration buffer of either phosphate buffered saline (PBS) or TRIS-HCI at a pH of 7.4 containing 8% polyethylene glycol (PEG) 8000. The virus was conditioned with PBS or TRIS-HCI buffer containing an appropriate PEG concentration to meet the criteria of the equilibration buffer. Samples were applied using either a 10 ml or a 150 ml superloop depending on the size of the membrane filter holder. After sample application, the column was washed with the same buffer that was used for initial equilibration and the virus finally eluted using PBS or TRIS-HCI pH 7.4 without PEG but supplemented with 0.4 M NaCl. A fresh stack of membranes was used for each chromatographic run. For the characterization of the SXC performance the 13 mm filter holder was operated with PBS buffer and at a flow rate of 0.5 mL min⁻¹. To evaluate subsequent polishing steps, larger amounts of SXC purified material were prepared. Therefore the 25 mm filter holder was used in combination with TRIS-buffer to reduce buffer exchanges between process units. Furthermore, during these runs a flow rate of 3 mL min⁻¹ was applied to increase throughput and minimize the process time. This was done three times, the elutions were pooled, aliquoted and frozen to -80°C until usage in a subsequent processing step. In parallel, freeze thaw stabilities in elution buffer were evaluated for 4 freeze and thaw cycles after SXC purification. In a further embodiment a second chromatography step was performed with size exclusion chromatography, ion exchange chromatography, hydrophobic interaction chromatography or pseudo-affinity chromatography, so that the virus is in the product fraction with overall virus yielding of >85% of the virus particles and an overall recovery of at least 10% and a degree of purity of at least 10% total protein reduction and 10% of the DNA content. The results are shown only for ORFV, but in principle are the same with other virus from family of poxviridae, for example genus Parapoxvirus.

### Ion exchange chromatography (IEX)

The IEX was tested for both, the capture and the polishing step. For that Sartobind S Pico devices were tested for cation exchange chromatography (CEX) and Sartobind Q and Sartobind STIC®-PA (STPA) were applied as anion exchangers (AEX) and salt-tolerant anion exchangers, respectively (all membrane devices were "pico"-scale obtained from Sartorius Stedim Biotech GmbH). For all IEX experiments, the columns were equilibrated with 20 mM TRIS-HCI pH 7.4 supplemented with 180 mM NaCl. Feed solutions were mixed with 20 mM Tris-HCL pH 7.4 to meet these conditions. Afterwards they were applied and the column was washed with 20 mM TRIS-HCI pH 7.4 supplemented with 180 mM NaCl until UV and light scattering signal reached the baseline. Using 2 M NaCl in 20 mM TRIS-HCI pH 7.4 the bound fraction was finally eluted. In case of the STPA membrane additionally 150 mM sodium phosphate were added during the elution. For all steps a flow rate of 1 mL min⁻¹ was applied. Clarified virus harvest was applied to the Q and the STPA membrane. SXC purified material was processed with all described IEX membranes. Cation exchange experiments were performed only one time, as the virus isoelectric point indicates a flow through of virus and impurities at the given buffer conditions. All anion exchange experiments were performed in triplicates.

### Hydrophobic interaction chromatography (HIC)

A membrane suitable for HIC was used for the polishing of SXC purified material, preferred a membrane with a macro-porous structure with pore size of > 3 µm for example Sartobind HICP Pico membranes (Sartorius Stedim Biotech GmbH). The column was equilibrated with 20 mM TRIS-HCI pH 7.4 supplemented with 180 mM NaCl and 1.7 M ammonium sulfate. SXC eluates were conditioned with an accordingly higher ammonium sulfate concentration to finally equal these amounts. After sample loading the membrane stack was washed under a maintained high salt concentration. The elution was achieved by removing the ammonium sulfate from the system using 20 mM TRIS-HCI pH 7.4 and 180 mM NaCl. HIC polishing steps were performed in triplicates using flow rates of 1 ml min⁻¹.

### Pseudo-affinity chromatography

Sulfated cellulose membrane adsorbers (SCMA) were used in a bind and elute mode. SCMA membranes were obtained as a paper sheet and punched to discs of 13 mm diameter. The discs were assembled into a 13 mm stainless steel filter holder with stacks comprising of 10 membrane layers. Both, clarified virus (capture) after step iii) and SXC purified material (polishing) after step v) was subjected to SCMA purification with triplicate runs for each case. Prior to chromatography the respective sample was mixed with 20 mM TRIS-HCI pH 7.4 in order to reduce its conductivity below 5 mS cm⁻¹. Namely, for all runs conductivities were in the range between 3 - 4.2 mS cm⁻¹. After equilibration of the membrane stack with 20 mM TRIS-HCI pH 7.4, the sample was applied and subsequently washed with equilibration buffer comprising of 20 mM TRIS-HCI pH 7.4. The elution was achieved using the same equilibration buffer supplemented with 2 M NaCl. A flow rate of 1 mL min⁻¹ was kept constant for all runs and phases.

### Size exclusion chromatography especially with the Capto™ Core 700 resin Capto core polishing

The Capto core concept is based on beads with a nonfunctionalized outer layer (without ligand) and a functionalized core with an attached ligand. The bead's pores in the outer layer, with an approximate exclusion limit of 700 kD, have been specifically designed to exclude large molecular entities such as viruses, large protein, or protein complexes from entering the internal space and interacting with the ligand, thereby enabling an efficient flow through purification step. As a possible polishing step Capto™ Core 700 resin (1ml, GE Healthcare Life Sciences) was tested in the flow through mode. The general method equals the conditions for the ion exchange membranes. The SXC purified material was adjusted to meet the conditions of the equilibration buffer, which was 20 mM TRIS-HCI pH 7.4 and 180 mM NaCl. After sample loading and washing with equilibration buffer the bound material was eluted using equilibration buffer with additional 2 M NaCl. Columns were regenerated by additional supplementation of the elution buffer with 0.5 M NaOH.

### Dynamic binding capacity determination

Dynamic binding capacity experiments were performed for all membranes operated in the bind-and-elute mode. Virus feed of a known concentration was loaded onto the column until complete breakthrough was observed based on light scattering detection. The amount of virus at 10% breakthrough was then calculated in relation to the maximum signal. Where necessary, the sample loading was stopped before 10% or 100% breakthrough occurred as the pressure exceeded the maximum operational pressure for the membrane or the system.

### Process performance including nuclease treatment

The best-performing stationary phases of the screening experiments were chosen for an evaluation of the overall DSP performance of the chromatographic set-up. For that, clarified virus was supplemented with nuclease for example Benzonase® nuclease and MgCl₂ at final concentrations of 500 U mL⁻¹ and 2 mM, respectively. Benzonase® degrades nucleic acid (DNA and RNA) and is useful in purification with high purity. The enzyme is a homodimer and needs Mg²⁺ (1-2 mM) as Cofactor. The incubation was done for 1 h at temperatures between 19 and 23°C. After incubation, EDTA was added to the mixture in a final amount of 5 mM, in order to block nuclease activity. Chromatographic purification was started directly after nuclease treatment without additional freeze/thawing. SXC and Capto core polishing were performed in series, as describes above. For the SXC, the 13 mm filter holder device and 20 mM Tris pH 7.4 supplemented with 180 mM NaCl as base buffer was used. For equilibration and wash, this buffer was supplemented with 8% PEG 8000 and for the elution instead of PEG 0.4 M NaCl were used. Clarified and nuclease digested harvest was mixed 3:4 with equilibration buffer containing 32% PEG 8000 to a final concentration of 8% PEG 8000.

### Size and isoelectric point of the purified virus from family of poxviridae, for example genus Parapoxvirus, especially ORFV

SXC purified virus were analyzed for their size and isoelectric point. Therefor SXC virus were subjected to size and surface potential measurements. For the size measurements a He-Ne laser (633 nm) and disposable semi-micro cuvettes were used in combination with a 90° light scattering detector angle. Dispersant refractive index and viscosity of the dispersant were set to 1.45 and 0.954 cP, respectively. For the surface potential measurement, the sample was mixed with 20 mM Tris at pH values between 3 and 13. The pH was checked adjusted to the respective value directly in advance of the measurement and reassessed once the measurement was finished. Folded capillary zeta cells were used to determine the viral zeta potentials at different pH values. Data analysis was done for example with the Malvern Zetasizer Software (version 7.12).

### Analytics of chromatographic fractions

Chromatographic fractions were analyzed for their content of infective virus as well as for the amount of protein and DNA. For the methods based on a bind and elute principle (SXC, AEX, HIC, SCMA) all fractions were considered including feed, flow through, wash and elution. For methods based on a flow through of the virus from family of poxviridae, for example genus Parapoxvirus, especially ORFV (CEX, Capto Core) the flow through and wash fractions were pooled and analyzed as an individual fraction.

### Flow-cytometric titration

Infective virus from one species from family of poxviridae, for example genus Parapoxvirus, especially ORFV were quantified using an automated flow cytometry-based approach. For that Vero cells were seeded in a 24-well format with 100.000 cells per well in a culture volume of 1 ml Dulbecco's Modified Eagle Medium (DMEN) supplemented with 5% fetal calf serum (FCS). DMEM is a widely used basal medium for supporting the growth of many different mammalian cells. Directly after seeding the cells, the infection of each well was performed using 100 µl of the respective chromatography sample, the medium blank or standard virus stock in the range of 1.5x10⁵ to 1x10⁷ infective particles. The cells were incubated for 16 hours, washed with PBS and harvested by use of Trypsin / EDTA.

Detached cells were supplemented with fetal calf serum (FCS) to stop the trypsin activity and transferred to a 96-U-bottom multiwell plate. The cells were washed two times by centrifugation at 400 g for 2 min, removing the supernatant and resuspending the pellet in 100 µl PBS. Finally, the cells were analyzed for example using a Guava® easyCyte HT system. Determined was the percentage of GFP positive (equals infected) cells compared to the total cell number. For the mCherry-genotype, yellow fluorescence was evaluated in order to quantify the amount of infected cells. The standard deviation was less than 10%.

### Total protein assay

The content of total protein was evaluated according to standard protocol for example using the Pierce™ BCA Protein Assay Kit. The performance was according to the instructions. In a 96-well microplate 25 µL for each sample were mixed with 200 µl working reaction and incubated at 37°C for 30 minutes. Afterwards, absorbance was measured at 562 nm using a plate reader. Bovine gamma globulin (kit contained) was used to make a standard calibration curve in the range of 20 - 2000 µg mL⁻¹ with a relative standard deviation of about 15%.

### dsDNA assay

The total dsDNA content was quantified for example using the Quant-iT™ PicoGreen® dsDNA Kit according to the manufacturer's instructions. Samples were analyzed in duplicates in a 96-well format using a plate reader for example the Cy-tation™ 3 plate reader. Each plate contained two standard calibration curves prepared from kit contained λ-DNA in the range of 0.025 ng m⁻¹l to 25 ng ml⁻¹ (low-range) and 1 ng ml⁻¹ to 1000 ng ml⁻¹ (high-range) respectively. Fluorescence excitation was at 480 nm and the emission intensity was measured at 520 nm with an assay standard deviation of <15%.

### Dynamic Light Scattering (DLS) measurement

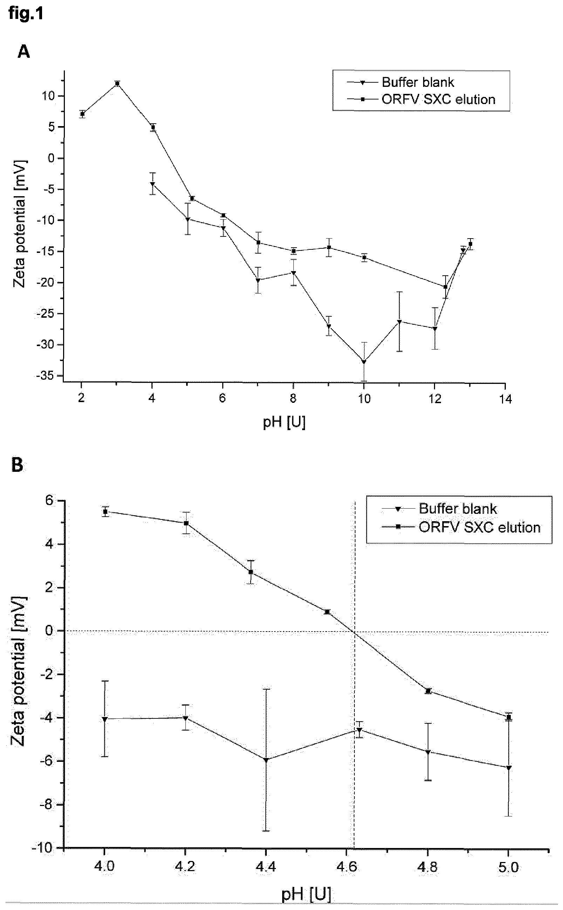

DLS measurements of purified virus from family of poxviridae, for example genus Parapoxvirus, especially ORFV showed a size distribution between 130 and 370 nm with two main size populations at about 150 ± 14 nm and 250 ± 32 nm. Zeta potential measurements detected a negative viral surface charge above pH values of 5 and a positive surface charge at a pH below or equaling 4 (figure 1A). A more detailed analysis of the pH range between 4 and 5 revealed the transition from positive to negative surface charge at a pH of 4.6, which was not seen for using buffer alone (figure 1B). The ORFV isoelectric point was determined with 4.6 under the given buffer conditions.

### Results for virus clarification

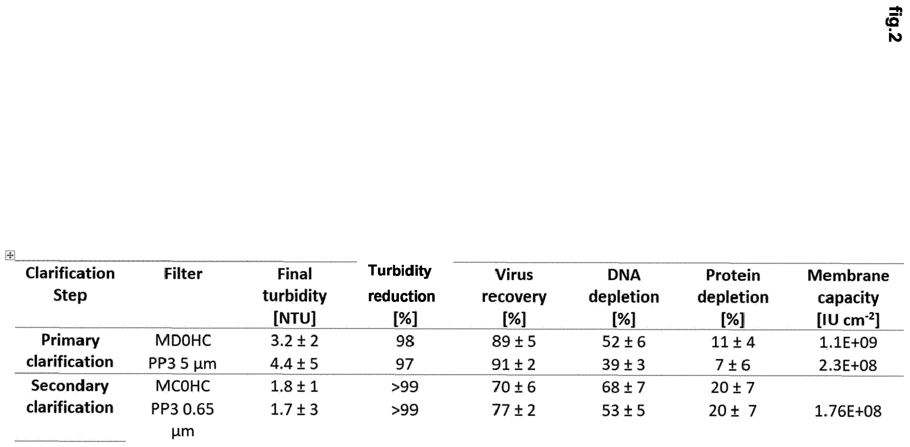

Centrifugal removal of whole cells or processing of non-centrifuged feed resulted in virus from family of poxviridae, for example genus Parapoxvirus, especially ORFV losses of about 50%, which is why recovery values were evaluated for clarified (centrifugation) feed only. Under these conditions, the performance of the first clarification was comparable for both evaluated filters with about 90% virus recovery (Figure 2). During the second clarification, the PP3 capsules performed equally with respect to the analytical error, yielding mean recoveries of 77% and 70% yield using the MC0HC devices. However, the Milistak® filters resulted in a slightly higher DNA depletion with 52% and 68% of the DNA being removed after primary and secondary clarification respectively. In contrast to that, for the PP3 capsules the final DNA depletion after second clarification accounted for only about 53%. The protein depletion was negligible for both filter combinations and resulted in a maximum removal of 20% of the total protein amount.

### Determination of the dynamic binding capacities

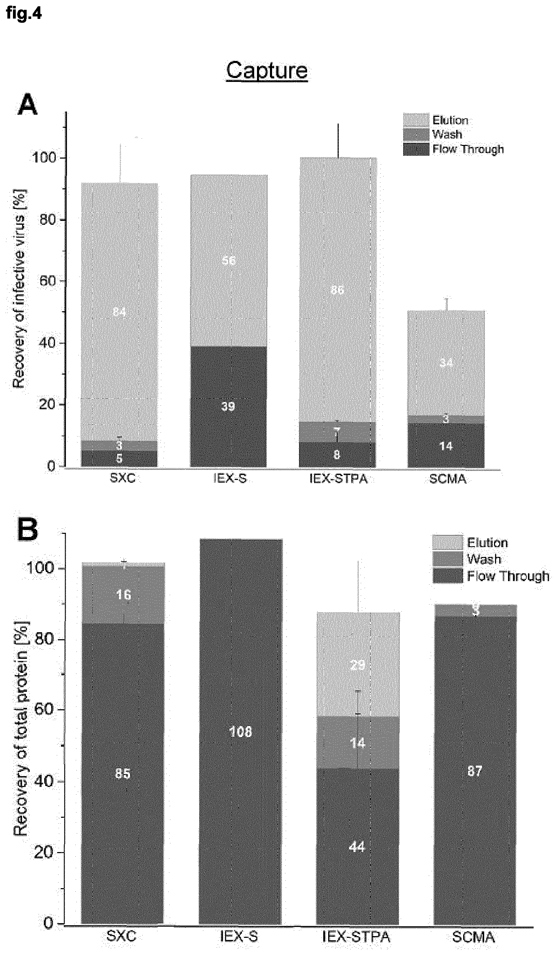

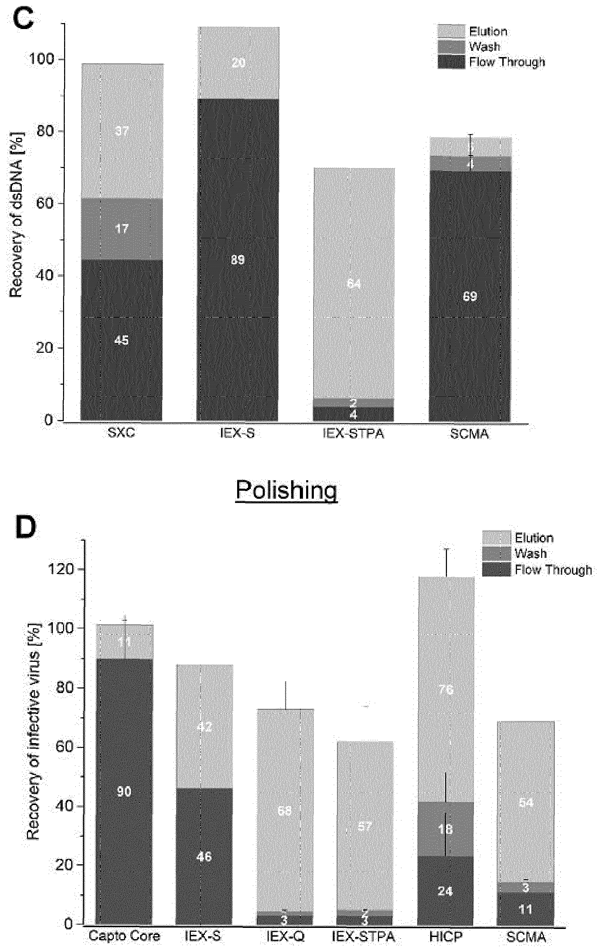

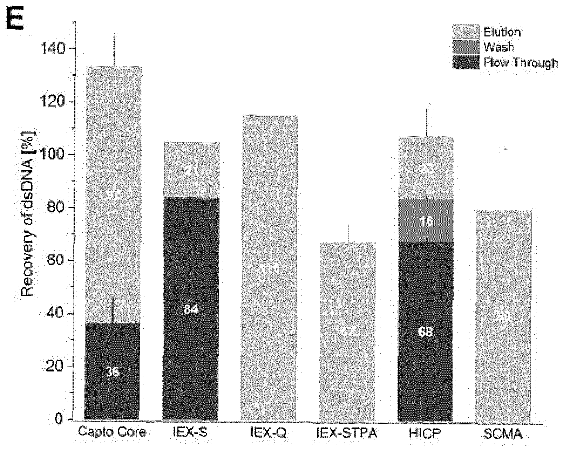

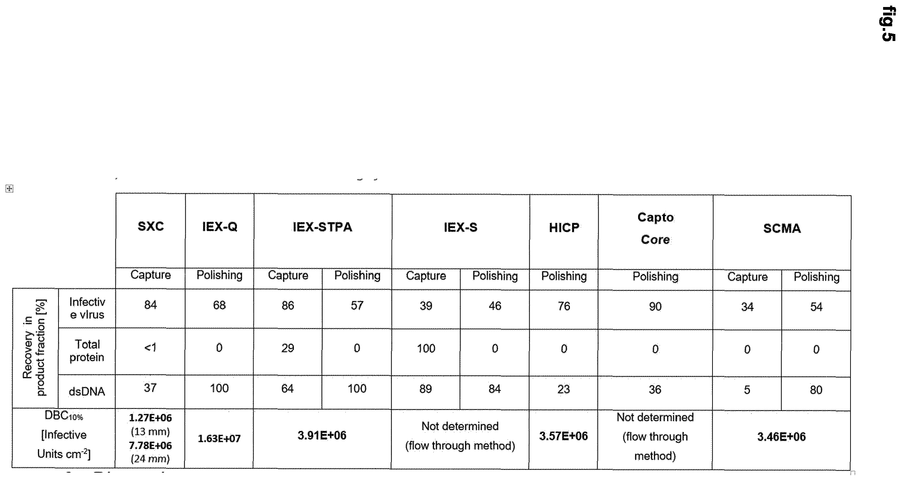

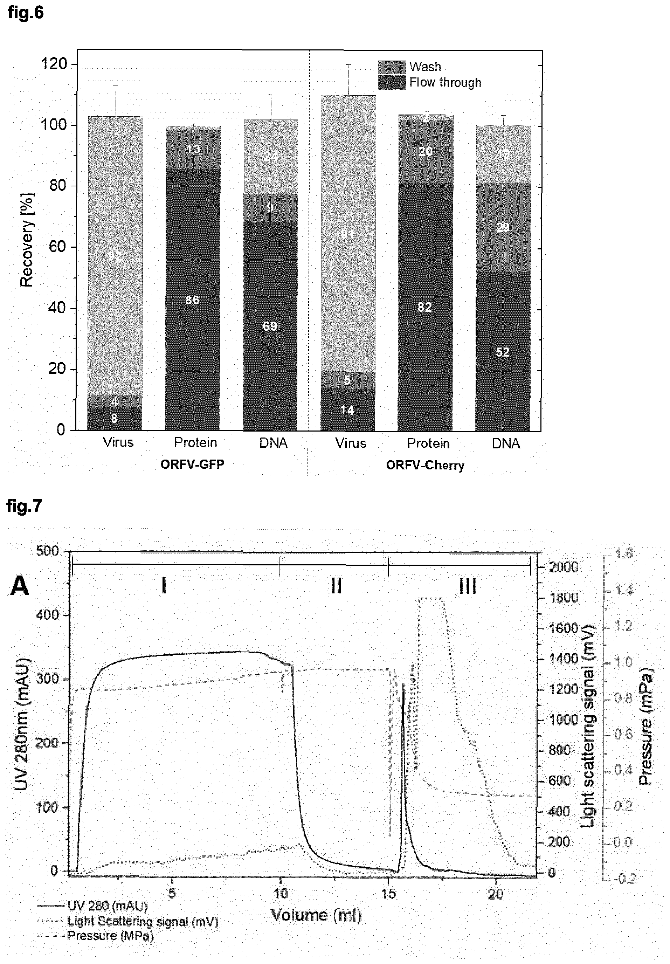

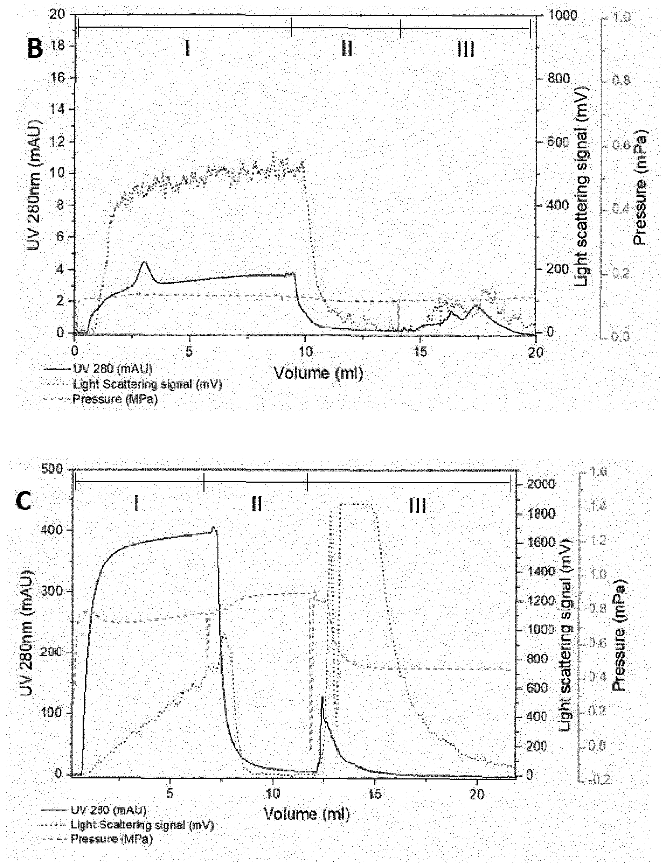

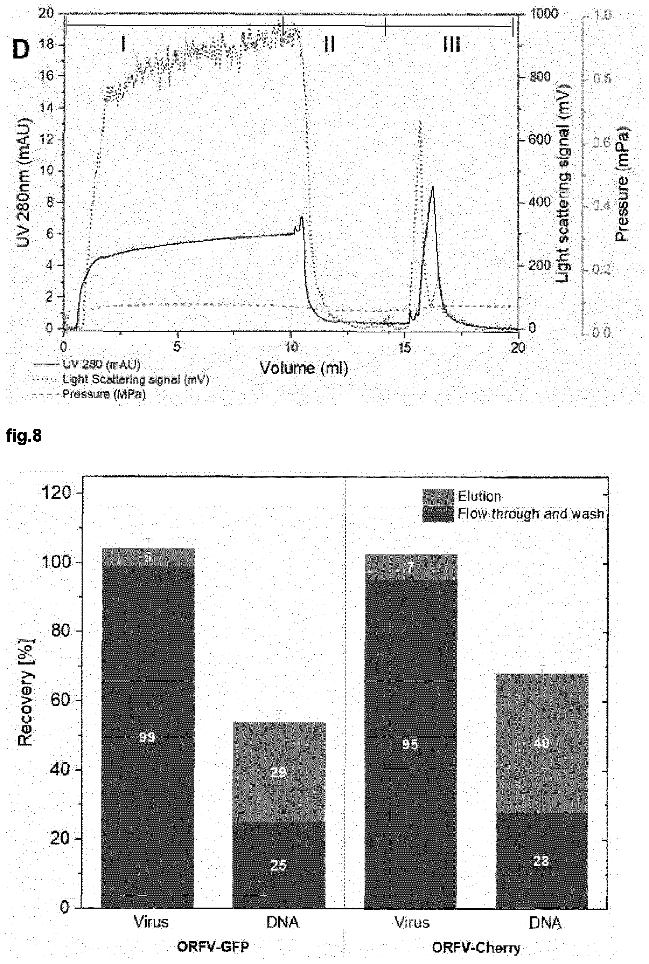

The dynamic binding capacities (DBC) was determined for all bind-and-elute methods at similar conditions using clarified virus. It was highest for the SXC using the 24 mm filter holder with 7.78E+06 IU cm⁻² and for IEX-Q membranes with 1.63E+07 IU cm⁻² (figure 4). For the 13 mm filter holder for SXC, a DBC_{10%} of 1.27E+06 IU cm^{- 2}could be determined (figure 5). In comparison to that, for the ORFV-Cherry a DBC_{10%} of 2.8E+06 IU cm⁻² was determined using the 13 mm filter holder. Comparable results could be determined for the IEX-STPA, HIC-P and SCMA membranes, with 3.91E+06, 3.57E+06 and 3.46E+06 IU cm⁻² respectively. A representative chromatogram is shown in figure 7 for the SXC (24 mm filter holder), indicating viral and contaminant breakthrough at different process stages. For the HIC-P at 10% breakthrough, also the pressure highly increased above 0.5 MPa, which is the operating limit given by the manufacturer. For the SCMA a constant breakthrough of 25% of the maximum signal was observed. As a result of that, there was no point were 10% breakthrough could be reached, instead the loading was stopped once the pressure increased above 1 MPa.

### Capture step - virus recovery and impurity removal

Of the four membranes tested for the capture step (SXC, IEX-S, IEX-STPA and SCMA) the best virus recovery was achieved for SXC and STPA with yields of 84% and 86%, respectively (figure 4, A). In contrast to that the recovery of infective virus particles in the product fraction was only 34% using the SCMA and 39% for the IEX-S membrane. For the SCMA only a total of about 51% of the virus could be found in all chromatographic fractions, whereas the remaining 49% could not be recovered. For the IEX-S method that the majority of virus was found in the elution fraction (56%) after application of 2 M NaCl and not as expected in the flow through. Concerning the protein removal, SXC and SCMA performed equally with a nearly complete protein depletion (figure 4, B). Contrary to that the complete protein load was recovered in the IEX-S protein fraction and about 29% were found in the IEXSTPA elution. The best unit operation to remove DNA during the capture step is the SCMA with only 5% of the initial DNA to be found in the product fraction and 95% of the DNA being removed during flow through and wash or remaining bound on the membrane (figure 4, C). For the SXC about 37% of the DNA were recovered in the elution. Most of the contaminating DNA remained in the product fractions of the IEX-S (89%) and the IEX-STPA membrane (64%). About 30% of the DNA were bound to the IEX-STPA membrane and could not be eluted in any of the fractions. Virus particles after SXC purification were stable for at least four freeze-thaw-cycles, without loss of infectivity.

### Size exclusion chromatography especially Capto Core as polishing step - virus recovery and impurity removal

During polishing, the highest virus recovery was achieved using size exclusion chromatography especially Capto Core with about 90% (figure 4, D). The second-best performing unit operation was the HICP membrane, resulting in virus yields of about 76% of the virus being found in the elution and about 40% losses in the flow through and wash. Similar to the capture step, for the cation exchange chromatography (CEX) membrane about half of the virus amount was found in the flow-through product fraction whereas the other half was bound to the column and eluted at 2 M NaCl. The tested anion exchange chromatography (AEX) membranes performed comparably with virus found in the product fraction of about 68% for the Q membrane and 57% for the IEX-STPA membrane, respectively. Notably a distinct amount of virus could not be eluted at 2 M NaCl with about 30% remaining on the Q device and about 40% on the IEX-STPA membrane. The SCMA performed better than during capture with less virus being retained on the device. However, overall recovery is only about 70%, thereof 54% are found in the product fraction.

For none of the applications used for polishing, proteins could be detected in any fraction. The most efficient DNA reduction was achieved using the Capto Core and the HICP membrane with 36% and 23%, respectively, of the applied DNA remaining in the product fraction (figure 4, E). For all other applications, the majority of contained DNA was yielded along with the virus with 84% (S-membrane), >100% (IEX-Q), 67% (IEX-STPA) and 80% (SCMA) being left. For the Q, IEX-STPA and SCMA applications no DNA was found during flow-through and wash. Furthermore, for STPA and SCMA the material balance could not be closed with 33% (IEX-STPA) and up to 20% (SCMA) of the DNA remaining on the membranes.

The most promising combination of downstream unit operations, regarding virus recovery and impurity removal, was an initial capture step using the SXC followed by a Capto core polishing step. However, DNA levels in the remaining product were still above 20 ng ml⁻¹ and thus above regulatory limits. Thus, for increasing the DNA removal efficiency, an additional nuclease treatment was included.

### Process evaluation including nuclease treatment

The SXC as a capture step after nuclease treatment performed similarly for both evaluated virus genotypes with more than 90% virus recovery in the elution fraction (figure 6). The protein removal was above 98% in each case and the remaining DNA amount in relation to the feed was 19 and 24% for the GFP and the Cherry genotype, respectively. During SXC purification and DBC determination for ORFV-Cherry it was observed, that breakthrough already started directly after the beginning of the application, whereas ORFV-GFP was completely retained during early sample loading (figure 7, A and C).

### Size exclusion chromatography Polishing

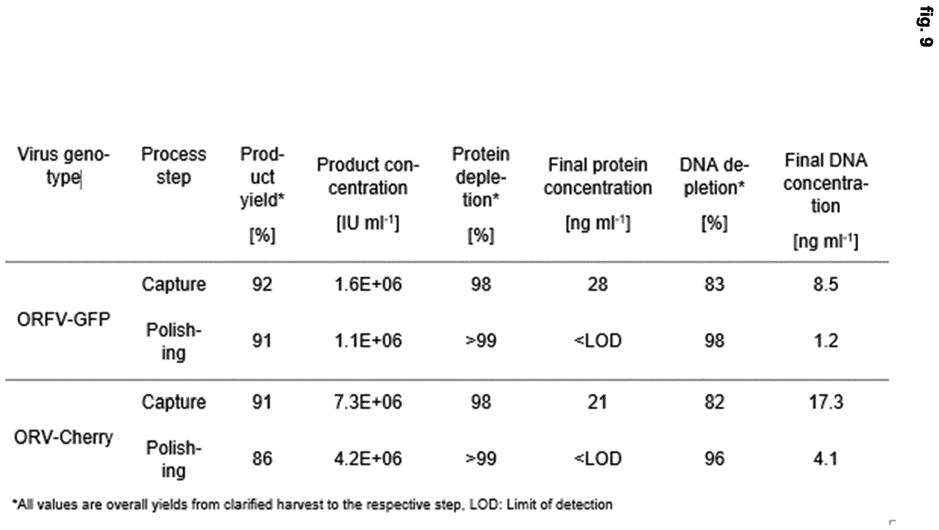

Using the capto core polishing, virtuell full virus recovery was possible with 99 ± 3% for the ORFV-GFP and 95 ± 1% for ORFV-Cherry being found in the product fraction, respectively. No protein was found in any of the chromatographic fractions. DNA was reduced during the polishing step for additional 75% and 72% for ORFV-GFP and ORFV-Cherry, respectively. In relation to 1 E+06 IU of virus, this led to final DNA contents in the product fraction of about 1 ng for the GFP genotype and about 2.2 for the Cherry genotype (figure 9).

Both evaluated ORFV genotypes shows different properties in terms of surface protein composition. While the GFP genotype, represents a rather native surface phenotype, with modifications only affecting the later protein expression in infected cells, the Cherry genotype carries two large membrane-bound proteins. Nevertheless, the overall influence on the process performance was negligible, with regard to analytical errors. Thus, it is clear that the process is robust for a large variety of different virus from family of poxviridae, for example genus Parapoxvirus, especially ORFV genotypes. This will enable further variations of the virus from family of poxviridae, for example genus Parapoxvirus, especially ORFV in order to improve efficiency, infectivity, or target specificity without the requirement for major adjustments of the downstream process. Dynamic binding capacities during the SXC are lower than previously reported for other viruses. A reason for that might be the rather large size of the virus from family of poxviridae, for example genus Parapoxvirus, especially ORFV compared to baculovirus particles or Influenza A. For ORFV-Cherry no DBC100% could be determined, instead, the sample application was limited by an elevating pressure. This might be caused by the large surface markers, which are additionally found on that genotype. On the one hand, these proteins might enhance the total binding capacity in contrast to the GFP genotype either by additional binding to other particles or by a higher retention due to sterical reasons. On the other hand, this further particle retention leads to an accelerated pore blocking on the membrane and thus leads to membrane fouling. In summary this is a robust and scalable downstream process for continuous clarification and purification of virus from family of poxviridae, for example genus Parapoxvirus. The process is suitable as platform technology and allows processing of virus from family of poxviridae, for example genus Parapoxvirus, especially ORFV with varying modifications and thus is suitable for different genotypes. Furthermore, the method can be used as a platform technology, to purify different virus genotypes, without major process changes. The yield of the clarification accounted for 70% and a maximum of 91% recovery was possible for the chromatographic set up. This resulted in an overall process yield of about 64%. Depending on the final dose, the levels of remaining process contaminants like host-cell protein and DNA were below regulatory limits. If desired, process unit operations are interchangeable, with probable outcomes found during the screening of possible stationary phases. The purified virus from family of poxviridae, for example genus Parapoxvirus, especially ORFV can be formulated in a pharmaceutical composition containing virus produced according to the invention. This pharmaceutical composition is for preventing for or treating human or veterinarian diseases. The purified virus is useful as vaccine against infectious diseases and cancer in human and animals. The purified virus is useful as viral vector for targeting tumor- or gene therapy or for immunostimulation or for immunostimulation.

The purified virus from this invention may be applied to patients orally, rectally, parenterally, intravenously, intramuscularly, subcutaneously, intracisternally, intravaginally, intraperitoneally, intravascularly, intrathecally, intravesically, topically, locally (powder, ointment or drops), or in spray form (aerosol). Pharmaceutically acceptable compositions of purified virus are available as salts, esters, amides or "prodrugs", provided that reliable medical evaluations do not indicate exceeding toxicity, irritations or allergic reactions when said compositions are applied. The term "prodrug" refers to compounds that are transformed for the sake of an improved reception, e.g. by hydrolysis in the blood. Pharmaceutical forms for the local application of the vaccine resulting from this invention include balms, powders, sprays, and inhalants. Under sterile conditions, the active component is mixed with a physiologically acceptable carrier and possible preservatives, buffers, diluents, and blowing agents, according to requirements.

**Abbreviations**

| | |
|---|---|
| AEX | Anion exchange chromatography |
| CEX | Cation exchange chromatography |
| DLS | Dynamic Light Scattering |
| HIC | Hydrophobic interaction chromatography |
| IEX | Ion exchange chromatography |
| PEG | Polyethylenglycol |
| SCMA | Sulfated cellulose membrane absorber |
| SXC | Steric exclusion chromatography |
| DBC | Dynamic binding capacity |
| GFP | Green fluorescence protein |
| IU | Infective Units forming units |

### List of Figures

**Fig**. **1** **A** shows the results of zeta potential measurements. We detected a negative viral surface charge above pH values of 5 and a positive surface charge at a pH below or equaling 4. **B** shows a more detailed analysis of the pH range between 4 and 5 revealed the transition from positive to negative surface charge at a pH of 4.6, which was not seen for using buffer alone. The virus' isoelectric point was determined with 4.6 under the given buffer conditions.
**Fig. 2** shows a table with summary of the clarification results, relative virus recoveries and protein and DNA depletion.

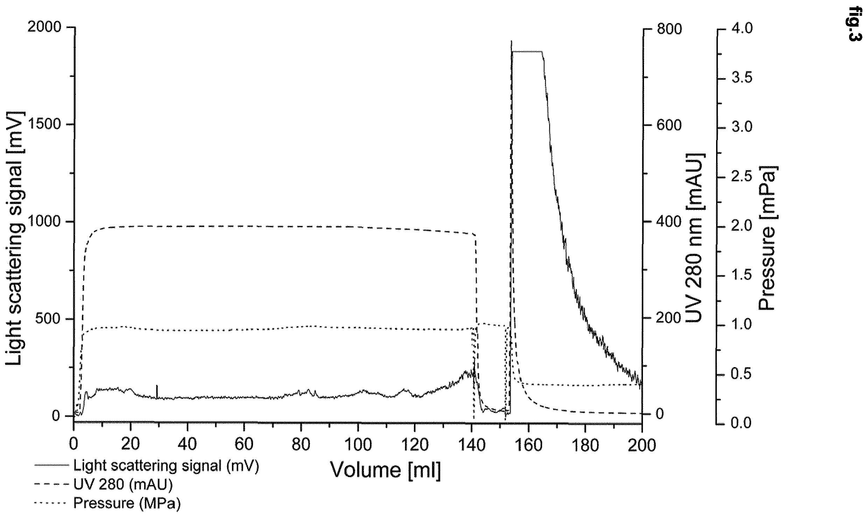

**Fig. 3** shows a representative chromatogram for the SXC (24 mm filter holder), indicating viral and contaminant breakthrough at different process stages. For the HIC-P at 10% breakthrough, also the pressure highly increased above 0.5 MPa, which is the operating limit given by the manufacturer. For the SCMA a constant breakthrough of 25% of the maximum signal was observed. As a result of that, there was no point were 10% breakthrough could be reached, instead the loading was stopped once the pressure increased above 1 MPa.
   The dynamic binding capacities was determined for all bind-and-elute methods at similar conditions using clarified virus from family of poxviridae and was highest for the SXC using the 24 mm filter holder with 7.78E+06 IU cm⁻² and for IEX-Q membranes with 1.63E+07 IU cm⁻². For the 13 mm filter holder for SXC, a DBC_{10%} of 1.27E+06 IU cm⁻² could be determined, less than for the larger device. Comparable results could be determined for the IEX-STPA, HIC-P and SCMA membranes, with 3.91E+06, 3.57E+06 and 3.46E+06 IU cm⁻² respectively.
**Fig. 4** **A** shows the results of the four membranes tested for the capture step (SXC, IEX-S, IEX-STPA and SCMA). The best virus recovery was achieved for SXC and STPA with viral yields of 84 and 86%, respectively. **B** shows the protein removal during capture. SXC and SCMA performed equally with a nearly complete protein depletion. **C** displays the SCMA as the best unit operation to remove DNA during the capture step with only 5% of the initial DNA to be found in the product fraction and 95% of the DNA being removed during flow through and wash or remaining bound on the membrane. **D** shows the results of polishing step using. The highest virus recovery was achieved with about 90% of the virus in the product fraction for the Capto core. **E** shows the most efficient DNA reduction during polishing was achieved using the Capto core and the HICP membrane with 36% and 23% of the applied DNA remaining in the product fraction.
**Fig. 5** shows a table with summary of the results of the screening of including the relative virus recovery, protein and DNA removal as well as the determination of the dynamic binding capacities (DBC).
**Fig. 6** shows the results from chromatographic purification with SXC capture after nuclease treatment. The SXC as a capture step performed similarly for both evaluated ORFV genotypes with more than 90% virus recovery in the elution fraction.
**Fig. 7** shows characteristic chromatograms for the capture and polishing steps for both ORFV genotypes (GFP, A-capture, B-polishing; Cherry, C-capture, D-polishing). The ORFV-Cherry breakthrough already started directly after starting the application (C), whereas ORFV-GFP was completely retained during early sample loading (A).
**Fig. 8** shows relative recoveries of virus from family of poxviridae and DNA during Capto core purification. Values depict the amount of virus being found in the individual fractions during the polishing step in relation to the amount applied to the Capto core column. The fractions flow through and wash were mixed as a combined product fraction, displayed by "flow through". Error bars represent technical triplicates. No protein was found in any of the polishing fraction.
**Fig. 9** shows a table including the results from the process evaluation using nuclease treated ORFV-GFP and ORF-Cherry. Shown are both, final concentrations in the purified product, as well as relative ORFV recovery and depletion values for the product and the two process contaminants evaluated.

All the results are shown only for ORFV as an example, but in principle are the same with other virus from family of poxviridae, for example genus Parapoxvirus. The invention is not limited to ORFV.

## Claims

1. Method for production of virus from family of poxviridae, comprising the following steps:
i) Providing cell culture with cells susceptible for virus from family of poxviridae
ii) Infection of cells from step i) with one species of virus from family of poxviridae to get infected cells
iii) clarification of virus from infected cells from step ii) by filtration, centrifugation, extraction, precipitation or expanded bed adsorption chromatography to remove particles ≥ 1µm and obtain a cell-free virus solution with at least 70% virus recovery
iv) nucleic acid degradation to remove nucleic acids in cell-free virus solution from step iii) with nuclease treatment to get a virus solution with degraded nucleic acid
v) purification of virus from solution of step iv) in at least one chromatographic step with steric exclusion chromatography (SXP) so that the virus is in the product fraction with virus capture yielding above 50% compared to the amount of virus in solution of step iii).

2. Method for production of virus from family of poxviridae according to claim 1 wherein the cells susceptible for virus from family of poxviridae in step i) are eukaryotic cells.

3. Method for production of virus from family of poxviridae according to claim 1 or 2 wherein the cells susceptible for virus from family of poxviridae in step i) are Vero-cells.

4. Method for production of virus from family of poxviridae according to one of the preceding claims wherein the infection of cells in step ii) occurs with virus of genus Parapoxvirus, especially ORFV.

5. Method for production of virus from family of poxviridae according to one of the preceding claims wherein the infection of cells in step ii) occurs with natural virus isolates or recombinant virus from family of poxviridae.

6. Method for production of virus from family of poxviridae according to claim 5 wherein the recombinant virus carrying at least one viral antigen from other viruses or at least one antigen typical for Aujeszky disease, Borna disease, Influenza or classical swine fever.

7. Method for production of virus from family of poxviridae according to claim 5 wherein the recombinant virus carrying the human CD4 surface markers or carrying at least one fluorescence gene.

8. Method for production of virus from family of poxviridae according to one of the preceding claims wherein the clarification in step iii) occurs by filtration with a sequence of depth filters with pore sizes between 1 - 10 µm to retain larger components of the harvest and allow passage of the virus.

9. Method for production of virus from family of poxviridae according to one of the preceding claims wherein step v) includes a second chromatographic step to remove proteins in the virus solution after step iv) with size exclusion chromatography, ion exchange chromatography, hydrophobic interaction chromatography or pseudo-affinity chromatography, so that the virus is in the product fraction with overall virus yielding of >85% of the virus particles compared to the amount of virus in solution of step iii) and an overall recovery of at least 10% and a degree of purity of at least 10% total protein reduction and 10% of the DNA content compared to the amount in solution of step iii).

10. Method for production of virus from family of poxviridae according to claim 9 wherein in step v) there is an additional filtration step between SXP and the second chromatography step.

11. Virus from family of poxviridae produced according to one of the preceding claims 1-10.

12. Pharmaceutical composition containing virus from family of poxviridae according to claim 11, produced according to one of the preceding claims 1-10, for preventing for or treating human or veterinarian diseases.

13. Use of virus from family of poxviridae produced according to one of the claims 1-10 as vaccine against infectious diseases and cancer in human or animals.

14. Use of virus from family of poxviridae produced according to one of the claims 1-10 as viral vector for targeting tumor- or gene therapy or for immunostimulation or used for immunostimulation in human or animals.
